(19) **Europäisches Patentamt / European Patent Office / Office européen des brevets**

(11) **EP 4 485 476 A1**

(12) **EUROPEAN PATENT APPLICATION**

(43) Date of publication:
**01.01.2025 Bulletin 2025/01**

(21) Application number: **23306096.1**

(22) Date of filing: **30.06.2023**

(51) International Patent Classification (IPC):
*G16H 50/20* (2018.01)  *G16H 50/30* (2018.01)

(52) Cooperative Patent Classification (CPC):
**G16H 50/20; A61B 5/0059; A61B 5/441; A61B 5/7267; G06T 7/0012; G16H 50/30;** G06T 2207/20076; G06T 2207/20084; G06T 2207/30088

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Pierre Fabre Dermo-Cosmétique**
**81500 Lavaur (FR)**

(72) Inventors:
• **Mac Carthy, Taig**
  **48001, Bilbao, Bizkaia (ES)**
• **Medela, Alfonso**
  **48001, Bilbao, Bizkaia (ES)**

(74) Representative: **Plasseraud IP**
**104 Rue de Richelieu**
**CS92104**
**75080 Paris Cedex 02 (FR)**

(54) **METHOD FOR PREDICTING THE ATOPIC DERMATITIS SEVERITY DYNAMICS OF AT LEAST ONE AREA OF A PATIENT**

(57)  The invention relates to a method implemented by computer means for predicting the atopic dermatitis severity dynamics of at least one area of a patient based on time-series images of the skin of said patient, said method including an inference phase comprising the following steps:

(a) segmenting each image to delineate at least one atopic dermatitis lesion in said image,

(b) predicting the evolution over time of at least one severity items of said segmented lesion, using a Bayesian state-space trained model for the prediction of each severity item.

Fig. 6

**Description**

FIELD OF THE INVENTION

The present invention relates to a method implemented by computer means for predicting the atopic dermatitis severity dynamics of at least one area of a patient based on time-series images of the skin of said patient.

BACKGROUND OF THE INVENTION

Atopic dermatitis (AD) or eczema is a prevalent chronic skin condition characterized by dry and itchy skin. The disease exhibits periods of flare-ups and remissions, which are often unpredictable, making treatment challenging and placing a burden on patients. Current treatments for mild and moderate AD involve the use of moisturizers on dry skin and anti-inflammatory creams or ointments (such as topical corticosteroids and calcineurin inhibitors) on inflamed skin. It is crucial to customize treatment approaches based on each patient's specific conditions to achieve optimal effectiveness, as treatment responses can vary among individuals and even within the same person. The development of personalized treatment strategies relies on the ability to predict future disease states for individual patients since AD symptoms fluctuate dynamically in a highly diverse manner.

The Harmonising Outcome Measures for Eczema (HOME) initiative suggested using the Eczema Area and Severity Index (EASI) as the primary measurement tool for assessing the clinical signs of eczema in clinical trials. The SCORing AD (SCORAD) and its objective component (oSCORAD) have also been validated as outcome measures, while other scoring systems like Six Area Six Signs AD (SASSAD) are commonly used in clinical practice. These instruments provide a single score that reflects the overall severity of eczema by combining scores from various severity items, including signs of intensity, subjective symptoms, and the extent of the affected area. Each severity item captures a distinct aspect of eczema severity and may exhibit its own patterns of change over time.

Such scoring systems may be complex and time-consuming to calculate for practitioners. The present document proposes a computer-implemented method for reliably predicting the atopic dermatitis severity dynamics.

SUMMARY OF THE INVENTION

To that aim, the present document proposes a method implemented by computer means for predicting the atopic dermatitis severity dynamics of at least one area of a patient based on time-series images of the skin of said patient, said method including an inference phase comprising the following steps:

(a) segmenting each image to delineate at least one atopic dermatitis lesion in said image,

(b) predicting the evolution over time of at least one severity items of said segmented lesion, using a Bayesian state-space trained model for the prediction of each severity item.

The severity dynamics of atopic dermatitis (AD) refers to the patterns and changes in the severity of AD symptoms over time. As mentioned above, AD is known for its fluctuating nature, with periods of exacerbation (flare-ups) and periods of remission. The severity dynamics encompass the variations in symptom intensity, duration, frequency, and extent of skin involvement throughout the course of the disease.

AD severity dynamics can vary widely among individuals, and even within the same person, as it is influenced by various factors such as genetic predisposition, environmental triggers, immune system responses, and individual patient characteristics. Understanding the severity dynamics is crucial for effective management and treatment of AD.

During flare-ups, the severity of AD symptoms tends to increase, with notable manifestations such as intense itching, redness, dryness, scaling, and skin lesions. These flare-ups can be triggered by factors like allergens, irritants, stress, weather changes, or infections. The severity dynamics may include rapid onset and progression of symptoms, leading to significant discomfort and impairment of the patient's quality of life.

Conversely, during periods of remission, AD symptoms subside or become less severe, resulting in improved skin condition and reduced symptoms. Remission phases can be spontaneous or occur with the aid of treatments or lifestyle adjustments.

The severity dynamics of AD can vary in terms of duration and frequency of flare-ups and remissions. Some individuals experience frequent and prolonged flare-ups with shorter remission periods, while others may have longer periods of remission between flare-ups. The severity of flare-ups can also differ, ranging from mild to severe.

Monitoring and understanding the severity dynamics of AD are crucial for tailoring treatment strategies to individual patients. It allows healthcare providers to adjust medications, lifestyle modifications, and preventive measures based on the specific needs of each patient, aiming to achieve optimal disease control, minimize symptoms, and improve overall quality of life.

In machine learning and statistical modeling, the inference phase and the training phase are two distinct stages of the model's lifecycle.

During the training phase, the model may be trained on a labeled dataset to learn patterns, relationships, and parameters that enable it to make predictions or perform a specific task. The training data consists of input examples and corresponding known output labels or target values. The model iteratively adjusts its internal parameters based on the training data to minimize the discrepancy between predicted outputs and the true labels. This process is often referred to as optimization or learning. The training phase aims to find the best set of model parameters that generalize well to unseen data and can make accurate predictions.

In other words, the training phase is the stage where a machine learning model learns patterns and relationships from labeled data to make accurate predictions or perform a specific task.

Once the model has been trained, said model may be used during the inference phase, also known as the deployment phase or prediction phase. In this phase, the trained model is used to make predictions or perform the desired task on new, unseen data. The inference phase involves applying the learned model to input data and obtaining output predictions or estimates based on the patterns and relationships it learned during training. Inference is typically the operational stage where the model is utilized to provide real-time predictions or perform tasks in production or practical applications.

In other words, the inference phase is the stage where the trained model is used to make predictions or perform tasks on new, unseen data in real-time or practical applications.

Time series images refer to a collection of images captured over a period of time, for example between 4 and 10 days, where each image represents a specific moment or frame in that sequence. These images are typically captured at regular intervals and are used to capture changes or variations that occur over time.

Said image may be a 2D image. A 2D image refers to an image that exists in two dimensions and consists of rows and columns of pixels, where each pixel represents a specific color or intensity value. A 2D image does not have depth or the perception of three-dimensional space.

Said image may represent a body part of said patient, such as head, trunk, arms, hands, legs, feet, genitalia, and/or skin close-up.

Segmentation of a skin lesion in an image refers to the process of identifying and delineating the boundaries of the lesion from the surrounding healthy skin in the image. It may involves separating the region of interest, i.e., the skin lesion, from the background.

Segmentation is an important step in medical image analysis, particularly in dermatology, as it allows for quantitative analysis and measurement of various features of the lesion, such as size, shape, and texture. It also plays a crucial role in computer-aided diagnosis and treatment planning.

The process of segmenting a skin lesion typically may involve at least some of the following steps:

- Preprocessing: The input image may undergo preprocessing steps like noise removal, contrast enhancement, and normalization to improve the quality and uniformity of the image.
- Image Enhancement: Techniques such as histogram equalization, filtering, and morphological operations may be applied to enhance the features and highlight the boundaries of the lesion.
- Feature Extraction: Image features relevant to skin lesion segmentation may be extracted. These may include color information, texture, and local image descriptors.
- Segmentation Algorithm: Various image segmentation algorithms may be employed, depending on the characteristics of the skin lesion and the specific requirements of the application.
- Post-processing: After obtaining an initial segmentation, post-processing steps may be applied to refine and improve the segmentation result. This may involve techniques like morphological operations (erosion, dilation), region-based filtering, or contour smoothing.

The above-mentioned segmentation algorithm may comprise thresholding, region-based segmentation (for example region growing or region splitting and merging), edge-based segmentation (for example edge detection, active contours (snakes), or level sets), machine Learning-based segmentation (for example using convolutional neural networks (CNNs)).

As mentioned above, in the context of evaluating the severity of atopic dermatitis (AD), various severity items or scoring systems may be used to assess the extent and intensity of the disease. These severity items are designed to provide a standardized and objective way to measure the severity of AD symptoms and track changes over time.

A Bayesian state space model (BSSM) is a statistical framework that combines state space modeling with Bayesian inference. It may be used to analyze time series data (here skin images over time) where the underlying process is assumed to have unobserved or latent states. BSSMs may be particularly useful when dealing with dynamic systems, such as biological processes, where the states evolve over time and are not directly observable.

In a BSSM, the system may be represented by two components: the state equation and the observation equation.

The state equation may describe the evolution of the latent states over time. It is typically represented as a set of equations that specify the transition dynamics of the states. The states can be continuous or discrete and can represent various aspects of the system being modeled.

The observation equation may relate the observed data to the underlying states. It describes the relationship between the latent states and the observed variables. The observed variables can be noisy measurements of the latent states, and the observation equation models the measurement process.

The goal of a BSSM may be to estimate the unknown states given the observed data and to infer the parameters of the state and observation equations. Bayesian inference may be used to obtain the posterior distribution of the states and parameters, taking into account both prior knowledge and the observed data.

Bayesian inference in BSSMs involves specifying prior distributions for the model parameters and using Bayes' theorem to update the prior beliefs based on the observed data, resulting in the posterior distribution. This posterior distribution may provide a probabilistic representation of the unknown states and parameters given the data, allowing for uncertainty quantification.

The time-period of said prediction may be comprised between 4 and 10 days.

The method according to the present document may have the following advantages over the prior art:

- Accurate Localization: Segmentation helps precisely delineate the boundaries of atopic dermatitis lesions in the skin image. This accurate localization allows for focused analysis and prediction of severity dynamics specifically within the identified lesions. It helps distinguish between affected and unaffected areas, reducing the potential for erroneous predictions due to the inclusion of unrelated regions.

- Objective and Quantitative Assessment: The use of computer-based methods provides an objective and quantitative assessment of atopic dermatitis severity dynamics. It removes potential subjectivity or variability associated with manual assessments by providing standardized measurements.

- Targeted Assessment: By segmenting the atopic dermatitis lesions, the method focuses on the specific areas of interest. It enables a more targeted and detailed analysis of the severity dynamics within the lesions, providing specific insights into the evolution of severity over time. This targeted assessment can be valuable for understanding disease progression, treatment response, and localized variations in severity.

- Non-Invasive and Convenient: The method relies on analyzing images of the skin, which is a non-invasive and convenient approach for patients. It eliminates the need for physical examinations or invasive procedures, making it more comfortable for patients during the assessment process.

- Enhanced Accuracy and Efficiency: Computer-based algorithms improves the accuracy of severity assessment compared to manual methods. Moreover, the use of machine learning techniques, such as Bayesian state-space trained models, enhances the predictive capabilities, allowing for more accurate and reliable predictions of severity dynamics. Segmentation also aids in enhancing the prediction performance of severity dynamics. By isolating the lesions, the method can account for the unique characteristics and dynamics specific to the affected areas. The Bayesian state-space trained model, trained specifically on the segmented lesion data, can better capture the complex temporal patterns and provide more accurate predictions of severity evolution.

- Real-Time Monitoring: The method enables real-time monitoring of atopic dermatitis severity dynamics. By predicting the evolution of severity items over time, clinicians can track the progression of the disease and make informed decisions regarding treatment strategies and adjustments.

- Personalized Treatment Approach: The method facilitates personalized treatment approaches by providing insights into the severity dynamics of specific areas in individual patients. This allows healthcare providers to tailor treatment plans based on the predicted severity dynamics, optimizing the effectiveness of interventions and potentially reducing the burden of the disease for patients.

- Research and Comparative Analysis: The segmented lesions and predicted severity dynamics provide valuable data for research and comparative analysis. Researchers can analyze the dynamics of atopic dermatitis within specific lesion regions, evaluate the effectiveness of different treatments, and explore correlations between severity evolution and various factors. This can contribute to the advancement of knowledge in the field and the development of new insights and strategies.

Step (b) may comprise predicting the evolution over time of multiple severity items of said segmented lesion, using a Bayesian state-space model for the prediction of each severity item.

Said method may comprise the following step:

(c) aggregating the prediction of each severity item made by each Bayesian state-space model to determine an aggregated severity score of said segmented lesion.

The aggregated severity score may be SCORAD and the severity items may comprise the extent and intensity signs of said segmented lesion. Said SCORAD may be based on each lesion of said patient.

The intensity signs may comprise dryness, redness, swelling, scabs or oozings, traces of scratching and/or thickening of said segmented lesion.

The severity items may further comprise subjective symptoms.

The subjective symptoms may comprise itching and sleep disturbance.

Of course, other aggregated severity score may be used, such as:

- Eczema Area and Severity Index (EASI)
- Patient-Oriented Eczema Measure (POEM)
- Investigator's Global Assessment (IGA)
- Three-Item Severity (TIS)
- Numerical Rating Scale (NRS)

The Bayesian state-space model for the prediction of the extent may be a binomial Markov chain.

The Bayesian state-space model for the prediction of each intensity sign extent may be an ordered logistic random walk.

The Bayesian state-space model for the prediction of each subjective symptom may be a binomial random walk.

Said Bayesian state-space models are defined below where the following notations are used in the present document:

- $y^{(k)}(t)$ is the score of interest of the k-th patient at time $t$. $k$ is dropped for models not taking the patient-dependence into account.
- $M$ is the maximum value that $y$ can theoretically take, i.e. $y \in [0,M]$.
- $y \sim \mathcal{N}(\mu, \sigma^2)$ denotes that $y$ is normally distributed with mean $\mu$ and variance $\sigma^2$.
- $y \sim \mathcal{N}^+(0, \sigma^2)$ denotes that y follows a half-normal distribution (normal distribution defined for positive values) of variance $\sigma^2$.
- $y \sim log\mathcal{N}(\mu, \sigma^2)$ denotes that $y$ is log-normal distributed, i.e. $\log(y) \sim \mathcal{N}(\mu, \sigma^2)$.
- $y \sim logit\mathcal{N}(\mu, \sigma^2)$ denotes that $y$ is logit normal distributed, i.e. $logit(y) \sim \mathcal{N}(\mu, \sigma^2)$.

Extent models: Binomial Markov chain

The extent model assumes that the body area can be subdivided into hundred patches, each with a probability, $p$ ($y = 1$) = $p$, of being classified as lesional, and that each patch has fixed transition probabilities between lesional and non-lesional states. The measurement is specified as a binomial distribution to count the number of lesional patches to produce the extent score, $A \sim B(100; p)$.

Two-state Markov chain models for latent dynamics

The transition is modeled from non-lesional to lesional or from lesional to non-lesional for any given patch of the skin with a two-state Markov chain characterized by the transition matrix

$$T = \begin{pmatrix} p_{00} & p_{01} \\ p_{10} & p_{11} \end{pmatrix} = \begin{pmatrix} 1 - p_{01} & p_{01} \\ p_{10} & 1 - p_{10} \end{pmatrix}$$

where

- $p_{10}$ is the transition probability from lesional to non-lesional states, i.e. the probability that a lesional patch is classified as non-lesional at the next step,
- $p_{11} = 1 - p_{10}$ is the probability that a lesional patch stays lesional and can be interpreted as a measure of eczema persistence, and
- $p_{01}$ is the transition probability from non-lesional to lesional states, i.e. the probability that a non-lesional patch is classified as lesional at the next step, and can be interpreted as a measure of eczema sensitivity to develop symptoms.

The probability, p(t), of a given patch being lesional is computed by

$$p(t+1) = p_{11}\, p(t) + p_{01}\big(1 - p(t)\big).$$

The state of a skin patch at time t is denoted by $x_t$ = (1 0) if it is non-lesional and $x_t$ = (0 1) if it is lesional. The predictions of the state at time $t + h$ is given by $x_{t+h} = x_t T^h$ with

$$T^h = \begin{pmatrix} 1-\pi & \pi \\ 1-\pi & \pi \end{pmatrix} + \lambda^h \begin{pmatrix} \pi & -\pi \\ -(1-\pi) & 1-\pi \end{pmatrix},$$

where $\lambda = 1 - p_{01} - p_{10}$ is one of the eigenvalues of T (the other one is 1) and $\pi = \frac{p_{01}}{p_{01}+p_{10}}$ characterizes the steady state (limiting) distribution, $x_\infty = (1 - \pi\ \pi)$, to which the Markov chain converges if the prediction horizon, $h$, is long enough. The value of $\lambda$ indicates the mobility of the Markov chain, i.e. how fast it converges to the steady state distribution, with $|\lambda| \to 0$ indicating faster convergence.

We assume that the probabilities, $p_{01}$ and $p_{10}$, are patient-dependent and that either one or both probabilities are time-dependent, given that the Markov chain converges to the steady state distribution, $\pi = \frac{p_{01}}{p_{01}+p_{10}}$, which is likely to evolve over a long enough time. We further assume that $p_{10} = 1 - p_{11}$ (where $p_{11}$ is a measure of eczema persistence) does not exhibit a strong time-dependence and that $p_{01}$ (a measure of eczema sensitivity to develop symptoms) dynamically changes due to endogenous and exogenous factors such as the skin barrier integrity and environmental stressors, respectively. We therefore parameterize the Markov chain by a time (t)- and patient (k)-dependent $\pi^{(k)}(t)$ and a patient-dependent $p_{10}^{(k)}$, from which we can derive

$$p_{01}^{(k)}(t) = p_{10}^{(k)} \frac{\pi^{(k)}(t)}{1-\pi^{(k)}(t)}.$$

<u>Priors of binomial Markov chain model</u>

The evolution of the extent, $A^{(k)}(t)$, for the $k$-th patient at time t is modelled with a binomial measurement model and latent Markov Chain model described above (the model equations include $\tilde{\pi}^{(k)}(t)$ since the upper bound of $\pi^{(k)}(t)$ conditioned on $p_{10}^{(k)}$ is $\frac{1}{1+p_{10}^{(k)}}$ where $p_{01}$ is between 0 and 1)

$$A^{(k)}(t) \sim \mathcal{B}(100, p^{(k)}(t)),$$

$$p^{(k)}(t+1) = p_{11}^{(k)} p^{(k)}(t) + p_{01}^{(k)}(t)(1 - p^{(k)}(t)),$$

$$p_{11}^{(k)} = 1 - p_{10}^{(k)},$$

$$p_{01}^{(k)}(t) = p_{10}^{(k)} \frac{\pi^{(k)}(t)}{1-\pi^{(k)}(t)},$$

$$\pi^{(k)}(t) = \frac{\tilde{\pi}^{(k)}(t)}{1+p_{10}^{(k)}},$$

$$logit\left(\tilde{\pi}^{(k)}(t+1)\right) \sim \mathcal{N}\left(logit\left(\tilde{\pi}^{(k)}(t)\right), \sigma^2\right),$$

with the priors,

$$\sigma \sim \mathcal{N}^+(0, (0.25\log(5))^2),$$

$$p_{10}{}^{(k)} \sim \text{logit}\mathcal{N}(\mu_{10}, \sigma_{10}{}^2),$$

$$\tilde{\pi}^{(k)}(t_0) \sim \text{logit}\mathcal{N}(-1,1),$$

$$\mu_{10} \sim \mathcal{N}(0,1),$$

$$\sigma_{10} \sim \mathcal{N}^+(0,1.5^2).$$

The priors are chosen to be weakly informative and translate to reasonable prior predictive distributions.

- The prior on $\sigma$ translates to an odd ratio increment for $\pi$ of at most 5. That is, $\pi(t) = 0.1 (OR(t) = 1/9)$ evolves up to $\pi(t+1) = 0.36$ $(OR(t+1) = 5 OR(t) \approx 0:56)$, which could be considered as an unusually important change.
- The prior for the initial condition, $\tilde{\pi}^{(k)}(t_0)$, at $t_0$ of the first data point is set to be slightly skewed toward 0, as high values of A are very unlikely.
- The priors on $\mu_{10}$ and $\sigma_{10}$ translate to an approximately uniform prior on $p_{10}{}^{(k)}$.

Intensity signs models: Ordered logistic random walk

It is assumed that measurements of the intensity signs are generated from an ordered logistic distribution, which is appropriate to describe ordinal random variables with only a few categories. An ordered logistic distribution is a logistic distribution with a location parameter (latent score), $y_{lat}^{(k)}(t)$, that is integrated between M cut-offs, $c(c_0 = 0 < c_1 < \cdots < c_{M-1})$,

$$y^{(k)}(t) \sim \text{OrderedLogistic}(y_{lat}^{(k)}(t), \mathbf{c})$$

$$\Leftrightarrow P\big(y^{(k)}(t) = i\big) = \begin{cases} 1 - \text{logit}^{-1}\big(y_{lat}^{(k)}(t) - c_0\big) \text{ if } i = 0 \\ \text{logit}^{-1}\big(y_{lat}^{(k)}(t) - c_{i-1}\big) - \text{logit}^{-1}\big(y_{lat}^{(k)}(t) - c_i\big) \text{ for } 0 < i < M - 1, \\ \text{logit}^{-1}\big(y_{lat}^{(k)}(t) - c_{M-1}\big) \text{ if } i = M. \end{cases}$$

$c_0 = 0$ is set without loss of generality since the ordered logistic distribution model is invariant by translation (adding $\lambda$ to $y_{lat}$ and c does not change the distribution).

The scale $y_{lat}$ depends on $c$, and is approximately $c_{M-1} - c_0 = c_{M-1}$, so we choose to express priors and the latent dynamic with $\tilde{y}_{lat}^{(k)}(t) = y_{lat}^{(k)}(t) * c_{M-1}$, a normalized version of $y_{lat}$.

We assume that $\tilde{y}_{lat}^{(k)}(t)$ follows a Gaussian random walk of variance $\sigma^2$.

$$\tilde{y}_{lat}^{(k)}(t+1) \sim \mathcal{N}(\tilde{y}_{lat}^{(k)}(t), \sigma^2),$$

and use the priors:

$$\delta \sim \mathcal{N}^+\left(0, \left(\frac{2\pi}{\sqrt{3}}\right)^2\right),$$

$$\tilde{y}_{lat}^{(k)}(t_0) \sim \mathcal{N}(\mu_0, \sigma_0{}^2),$$

$$\mu_0 \sim \mathcal{N}(0.5, 0.25^2),$$

$$\sigma_0 \sim \mathcal{N}^+(0, 0.125^2),$$

$$\sigma \sim \mathcal{N}^+(0, 0.1^2),$$

where $\delta$ are the differences between consecutive cutpoints, i.e. $\delta_i = c_{i+1} - c_i$ for $i \in [0, M - 1]$.

The priors are chosen to be weakly informative and translate to reasonable prior predictive distributions.

- The prior on $\delta$ translates to values less than the width of the standard logistic distribution allowing the possibility of $P$ ($y^{(k)}(t) = i) \approx 1$ (the standard deviation of the standard logistic distribution is $\frac{\pi}{\sqrt{3}}$).

- The prior on $\mu_0$ and $\sigma_0$ translate to an approximately uniform prior for the initial latent score, $y_{lat}^{(k)}(t_0)$ within the range of $y_{lat}$.

- The prior on $\sigma$ assumes it is possible to go from a state where $y = 0$ is the most likely outcome, to a state where $y = M$ is the most likely outcome, in two transitions.

Subjective symptoms models: Binomial random walk

The latent score, $y_{lat}^{(k)}(t)$, for subjective symptoms (discrete variables with a large number of categories) is assumed to follow a random walk on the logit scale, and the measurement is modelled by a binomial distribution whose success parameter, $p$, is allowed to vary with time,

$$y^{(k)}(t) \sim \mathcal{B}(M, p^{(k)}(t)),$$

$$\text{logit}(p^{(k)}(t + 1)) \sim \mathcal{N}(\text{logit}\left(p^{(k)}(t)\right), \sigma^2),$$

with the priors,

$$\sigma \sim \mathcal{N}^+(0, (0.25 \log(5))^2),$$

$$p^{(k)}(t_0) \sim \text{logit}\mathcal{N}(\mu_0, \sigma_0^2),$$

$$\mu_0 \sim \mathcal{N}(0,1),$$

$$\sigma_0 \sim \mathcal{N}^+(0, 1.5^2),$$

The priors are chosen to be weakly informative and translate to reasonable prior predictive distributions.

- The prior on $\sigma$ translates to an odd ratio increment for $p$ of at most 5. For instance, if p(t) = 0.1 $\left(OR(t) = \frac{1}{9} \approx 0.11\right)$, then the prior assumes it is unusual, but not impossible, that the next value is $p(t + 1) = 0.36$ ($OR(t + 1) = 5OR(t) = 5/9 \approx 0.56$).
- The priors on $\mu_0$ and $\sigma_0$ have a reasonable range and translate to a marginal prior for $p^{(k)}(t_0)$ that is approximately uniform.

Step (a), or segmentation step, may be performed by using a trained U-net model.

U-Net is a convolutional neural network (CNN) architecture used notably for image segmentation tasks.

The U-Net architecture is named after its U-shaped design, consisting of an encoding path (contracting path) and a decoding path (expanding path). The encoding path captures contextual information and reduces the spatial dimensions of the input image, while the decoding path recovers the spatial information and generates a segmentation map.

The key features of the U-Net architecture are as follows:

- Contracting Path: The encoding path consists of multiple down-sampling layers, typically implemented using convolutional and pooling operations. Each down-sampling layer reduces the spatial dimensions while increasing the number of feature channels, allowing the network to capture increasingly abstract and contextual information.

- Expanding Path: The decoding path is responsible for recovering the spatial details and generating a high-resolution segmentation map. It consists of up-sampling layers that increase the spatial dimensions while reducing the number of feature channels. The up-sampling layers may be accompanied by skip connections that concatenate feature maps from the corresponding levels in the encoding path. These skip connections help in preserving spatial details and combining them with contextual information.

- Skip Connections: U-Net's skip connections enable information flow between the encoding and decoding paths at multiple resolutions. By combining the high-resolution features from the encoding path with the up-sampled features in the decoding path, the network can effectively localize and segment objects with fine details.

- Symmetric Architecture: U-Net's symmetric architecture ensures that the spatial information lost during down-sampling is effectively recovered during up-sampling, allowing for accurate segmentation outputs.

Such architecture is able to capture both local and global contextual information while preserving fine-grained spatial details, making it particularly suitable for tasks requiring precise and accurate segmentation.

Resnet model, for example ResNet-34, may be used as a backbone for the U-Net.

In the context of the U-Net architecture, the backbone refers to the encoder part of the network. The backbone typically consists of pre-trained convolutional neural network (CNN) models that have been proven effective in various computer vision tasks.

The choice of backbone for U-Net depends on the specific requirements of the task at hand and the available pre-trained models. Such backbones for U-Net include VGG, ResNet, MobileNet or EfficientNet for example.

The residual connections in ResNet backbone help address the vanishing gradient problem and facilitate the learning of deeper representations.

These backbones are typically used in transfer learning scenarios, where the pre-trained weights of the backbone are initialized with weights learned from large-scale image classification tasks such as ImageNet. The backbone is then fine-tuned with the specific task of image segmentation using the U-Net architecture.

By using a pre-trained backbone as the encoder, U-Net can leverage the learned features from the large-scale classification task, which helps in extracting meaningful and high-level features from the input image. This aids in accurate and efficient segmentation of the target objects or regions.

The present document also concerns a computer program comprising instructions for implementing the above-mentioned method, when this program is executed by a processor.

The present document also concerns a non-transitory computer-readable recording medium on which is recorded a program for implementing the above-method, when said program is executed by a processor.

The present document also concerns a computer device comprising:

- an input interface to receive time-series images,

- a memory for storing at least instructions of the above-mentioned computer program,

- a processor accessing to the memory for reading the aforesaid instructions and executing then the above-mentioned method,

- an output interface to provide an information based on the prediction of step (b).

BRIEF DESCRIPTION OF THE DRAWINGS

Other features, details and advantages will be shown in the following detailed description and on the figures, on which:

- figure 1 schematically shows an example of a computer device according to the present document,
- figure 2 schematically shows an example of the method according to the present document,
- figure 3 illustrates (a) an original image of the skin of a first body part of patient and (b) the lesion surface segmentation masks onto said image,
- figure 4 illustrates (a) an original image of the skin of a second body part of patient and (b) the lesion surface segmentation masks onto said image,

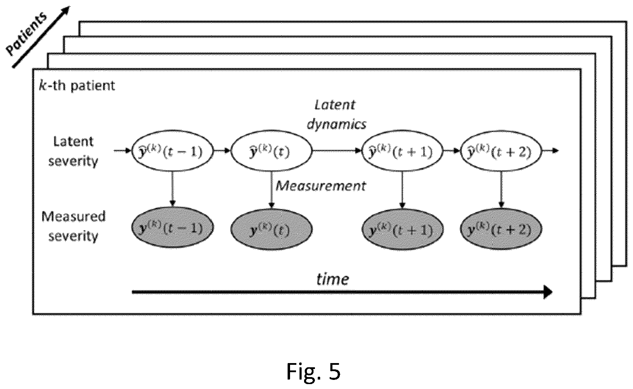

- figure 5 illustrates Bayesian state-space models according to the present document, where each model describes the dynamics of a latent severity (white ovals) and the measurement of the latent severity to obtain the recorded severity (grey ovals),

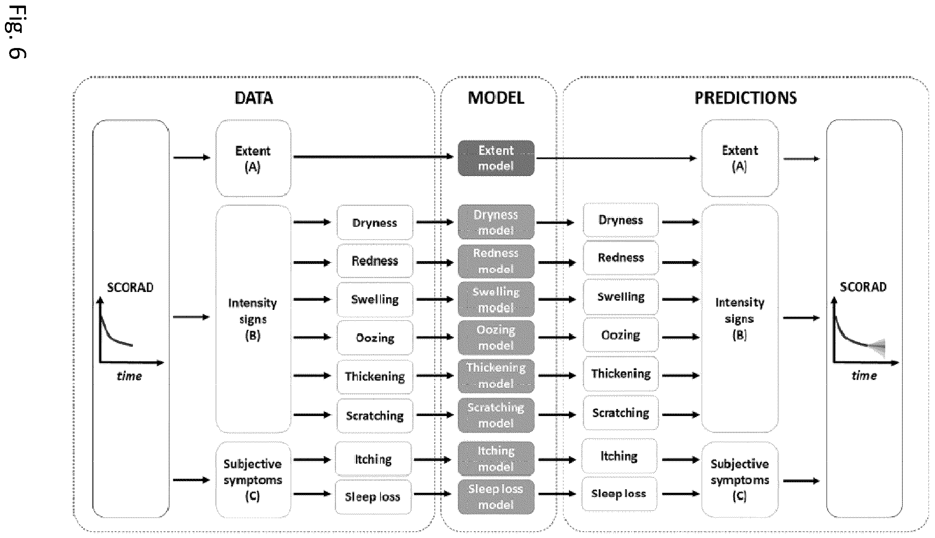

- figure 6 illustrate SCORAD prediction using nine models (colored rectangles), each of which corresponds to one of the nine severity items for SCORAD, are aggregated to provide predictions for SCORAD.

The annexed drawing includes meaningful colors. Although the present application is to be published in black and white, a colored version of the annexed drawing was filed before the Office.

DETAILED DESCRIPTION OF A PREFERRED EMBODIMENT

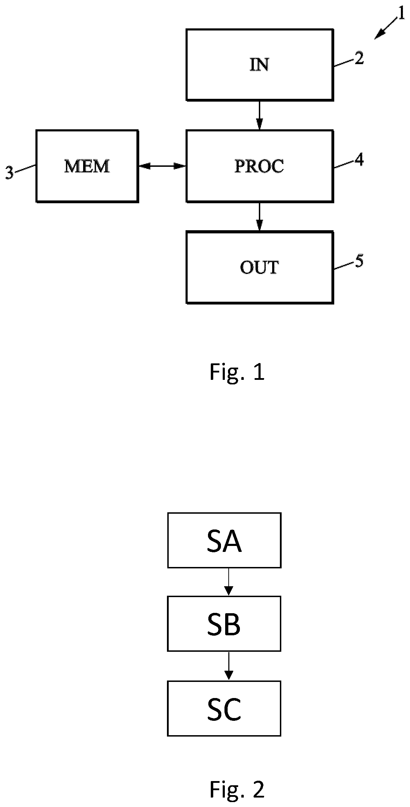

Figure 1 schematically shows an example of a computer device 1 according to the invention. Said computer device 1 comprises:

- an input interface 2,
- a memory 3 for storing at least instructions of a computer program,
- a processor 4 accessing to the memory 3 for reading the aforesaid instructions and executing the method according to the present document,
- an output interface 5.

The method according to the present document (as shown in figure 2) is a method implemented by computer means for predicting the atopic dermatitis severity dynamics of at least one area of a patient based on time-series images of the skin of said patient, said method including an inference phase comprising the following steps:

- segmenting (SA) each image to delineate at least one atopic dermatitis lesion in said image, using a trained segmentation model, more particularly a U-net model,
- predicting (SB) the evolution over time of severity items of said segmented lesion, using a Bayesian state-space trained model for the prediction of each severity item,
- aggregating (SC) the prediction of each severity item made by each Bayesian state-space model to determine an aggregated severity score of said segmented lesion.

- **Training and validation of the segmentation U-net model**

Datasets

Three annotated datasets were constructed to train and validate the performance of the lesion surface segmentation. The first two datasets comprise solely light-skinned patients (Fitzpatrick I-III), whereas the third dataset consists of images of sark skinned patent (Fitzpatrick IV-VI).

The first dataset is a dataset collected from online dermatological atlases that consist of 604 images that belong to light-skinned patients, of which one third are children, suffering from AD, with lesions present on different bodyparts. The dataset contains the following percentage of body zones: head (22%), trunk (11%), arms (23%), hands (9%), legs (16%), feet (8%), genitalia (3%), full body (1%), and skin close-up (7%). The dataset contains a substantial variety of clinical images taken from different angles, distances, light conditions, body parts, and disease severity. The images have a minimum size of 260 x 256 pixels, an average size of 667x563 pixels, and a maximum size of 1772x1304 pixels.

The second dataset was built for testing purposes. The dataset was gathered from several dermatological atlases publicly available and contains a total number of 367 images that belong exclusively to light-skinned patients. Each image was reviewed by a physician to approve the inclusion of the image in the dataset. Duplicates or very similar images were removed, and noother data sampling technique was applied. The dataset contains images of children and adults with great variability in angles, distances, light conditions, body parts, and disease severity. The second dataset contains the following percentage of body zones: head (35%), trunk (20%), arms (18%), hands (7%), legs(13%), feet (2%), genitalia (2%), and skin close-up (3%). The images have a minimum size of $313\times210$ pixels, an average size of 574x537 pixels, and a maximum size of 2848x3252 pixels.

The third dataset is a dataset collected from online dermatological atlases that contain photos of children and adult patients with Fitzpatrick IV-VI- skin types suffering from AD. It is composed of 112 images with a minimum size of 200x204 pixels, an average size of 766x695 pixels, and a maximum size of 3,024x4,032 pixels. The dataset contains the following percentage of body zones: head (41%), trunk (10%), arms (17%),hands (8%), legs (13%), feet (3%), and skin close-up (8%). The goal of including this dataset in the study was to gather preliminary evidence of the efficiency of the algorithm in dark skin.

### Ground truth labels

The corresponding ground truths of each dataset were prepared by nine experts, three for each dataset, who treat patients with AD in their daily practice, to reduce variability by combining their results. The experts annotated the images without more context than the images. They had to draw a mask over the lesion. The ground truth labels were obtained for lesion segmentation by averaging the masks of the three annotators. The mean was chosen over the median because it is the statistical measure that gets the best results for generating ground truth labels from multiannotator ordinal data.

### Data preprocessing

Images were resized to 512x512, and pixel values scaled between 0 and 1. In addition, images in which the disease was too small in the picture were cropped, focusing on the disease. Ground truth labels were obtained from averaging the results as explained in the previous section. With regard to lesion surface masks, the average mask was computed, resulting in a grayscale image in the range 0-255. A pixel intensity threshold of 155 was applied to obtain a binary mask that was used as the ground truth. Images were finally normalized to the range 0-1.

### Evaluation metrics

Pixel accuracy, Area Under the Curve (AUC), Intersection over Union (IoU), and F1-score metrics were the preferred metrics to evaluate the segmentation model.

### Experimental setup

Two main experiments were carried out: one with images containing only light skin and another adding a small number of darkskin images in the training set. In the firs experiment, the first data set was used for training and the second and third datasets were used for testing. A six-fold cross-validation strategy was carried out to train the models. The models trained on the different folds were tested on both test sets, and the results were averaged over the folds to reduce the variance and biais.

The second experiment was built to better understand the performance of the network on dark skin when including a tiny fraction of dark-skinned patient images in the training set. In this experiment, the first dataset, the second dataset and a subset of the third dataset were used for training and the rest for testing. The training and test subsets of the third dataset were obtained with a three-fold cross-validation strategy. This means that the training set was composed of 971 light-skinned patient images, first and second dataset combined, and 75 dark-skinned patient images (third dataset), which is a tiny fraction of the total images (8%). The dark skin test set was composed of the remaining 37 images of the third dataset. This split was done three times (three-fold), including different images in the training and test set, to obtain more reliable results.

### Results

The AUC, IoU, and F1 for light-skin were 0.93, 0.64, and 0.75, respectively, whereas the results on those metrics were 0.83, 0.32, and 0.42, respectively, for dark skin.

When training in a small subset of dark skin images (experiment 2), it appears that the results are further improved (0.41 for IoU and 0.33 for F1).

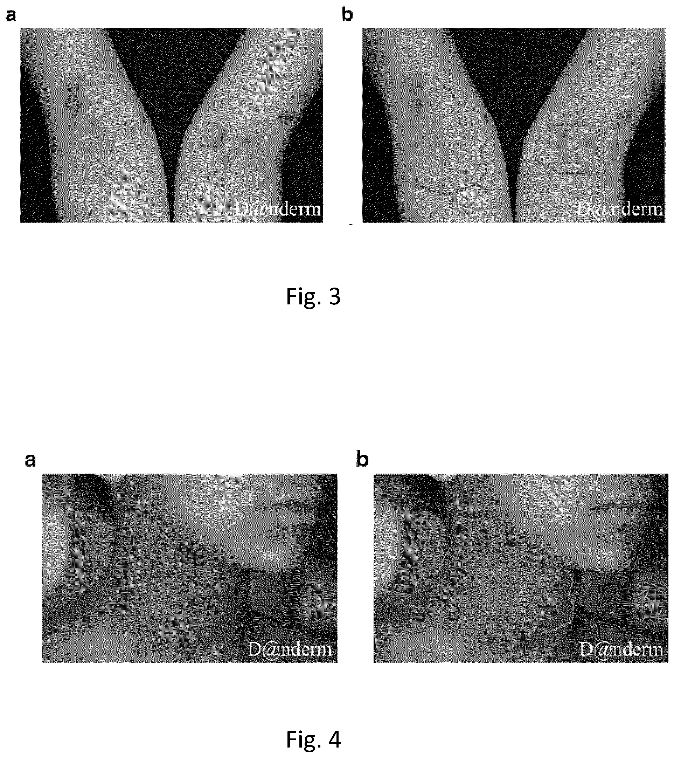

Figures 3 and 4 illustrate segmented images, for two different skin images.

### - **Training and validation of the Bayesian state-space models**

### Models overview

A collection of nine machine learning models (Bayesian state-space models) is used to predict each of the nine severity items for SCORAD. Predictions from the nine models are aggregated to produce predictions for SCORAD by assuming independence of the severity items. The latent dynamics and measurement distributions of the state-space models were tailored to each severity item. The latent dynamics was modelled for the extent as a Markov chain that describes how a small "patch" of the skin transitions from non-lesional to lesional and vice versa. A binomial distribution was used to count the numbers of lesional patches, that is the extent. We assumed random walk latent dynamics for the intensity signs and subjective symptoms in the absence of precise insights about the item-specific data-generating mechanisms. The measurement of intensity signs from the latent scores was described by an ordered logistic measurement distribution and that of subjective symptoms by a binomial distribution. Parameters of the measurement distributions and the variance of the random walk latent dynamics were shared between patients, while parameters of the Markov chain latent dynamics were made patient-dependent with hierarchical priors. Priors were chosen to be weakly informative. Missing values were treated as an absence of measurement in the state-space models.

Model inference was performed using the Hamiltonian Monte Carlo algorithm.

Model validation

The predictive performance of the models was evaluated in a forward-chaining setting, where the models were trained every four days. That is, the models were first trained on the first day's data of a patient and tested them using their data over the next four days, then trained the models on the first five days' data and tested them on the next four days' data, etc.

The probabilistic predictions of SCORAD and its nine severity items may be evaluated using a logarithmic scoring rule, the log predictive density (lpd). An accuracy metric may also be computed for SCORAD predictions.

**Claims**

1. A method implemented by computer means for predicting the atopic dermatitis severity dynamics of at least one area of a patient based on time-series images of the skin of said patient, said method including an inference phase comprising the following steps:

   (a) segmenting each image to delineate at least one atopic dermatitis lesion in said image,
   (b) predicting the evolution over time of at least one severity items of said segmented lesion, using a Bayesian state-space trained model for the prediction of each severity item.

2. The method according to preceding claim, wherein step (b) comprises predicting the evolution over time of multiple severity items of said segmented lesion, using a Bayesian state-space model for the prediction of each severity item.

3. The method according to preceding claim, wherein said method comprises the following step:
   (c) aggregating the prediction of each severity item made by each Bayesian state-space model to determine an aggregated severity score of said segmented lesion.

4. The method according to preceding claim, wherein the aggregated severity score is SCORAD and the severity items comprise the extent and intensity signs of said segmented lesion.

5. The method according to preceding claim, wherein the intensity signs comprise dryness, redness, swelling, scabs or oozings, traces of scratching and/or thickening of said segmented lesion.

6. The method according to one of the preceding claims, wherein the time-series images are captured over a period of time comprised between 4 and 10 days.

7. The method according to one of the preceding claims, wherein the time-period of said prediction is comprised between 4 and 10 days.

8. The method according to any of claims 4 to 7, wherein the Bayesian state-space model for the prediction of the extent is a binomial Markov chain.

9. The method according to any of claims 4 to 8, wherein the Bayesian state-space model for the prediction of each intensity sign extent is an ordered logistic random walk.

10. The method according to any of claims 6 to 9, wherein the Bayesian state-space model for the prediction of each subjective symptom a binomial random walk.

11. The method according any of preceding claims, wherein step (a) is performed by using a trained U-net model.

12. A computer program comprising instructions for implementing the method according to one of claims 1 to 11, when this program is executed by a processor.

13. A non-transitory computer-readable recording medium on which is recorded a program for implementing the method according to one of claims 1 to 11, when said program is executed by a processor.

14. A computer device (1) comprising:

    - an input interface (2) to receive time-series image,
    - a memory (3) for storing at least instructions of a computer program according to claim 12,
    - a processor (4) accessing to the memory (3) for reading the aforesaid instructions and executing then the method according to one of claims 1 to 11,
    - an output interface (5) to provide an information based on the prediction of step (b).

Fig. 1

Fig. 2

a

b

Fig. 3

a

b

Fig. 4

Fig. 5

Fig. 6

**Europäisches Patentamt**
**European Patent Office**
**Office européen des brevets**

# EUROPEAN SEARCH REPORT

**Application Number**

EP 23 30 6096

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SUHENDRA RIVANSYAH ET AL: "A Novel Approach to Multi-class Atopic Dermatitis Disease Severity Scoring using Multi-class SVM", 2019 IEEE INTERNATIONAL CONFERENCE ON CYBERNETICS AND COMPUTATIONAL INTELLIGENCE (CYBERNETICSCOM), IEEE, 22 August 2019 (2019-08-22), pages 35-39, XP033636411, DOI: 10.1109/CYBERNETICSCOM.2019.8875693 [retrieved on 2019-10-17] * the whole document * | 1-14 | INV. G16H50/20 G16H50/30 |
| Y | HELSKE JOUNI ET AL: "bssm: Bayesian Inference of Non-linear and Non-Gaussian State Space Models in R", THE R JOURNAL, vol. 13, no. 2, 4 January 2017 (2017-01-04), page 471, XP093112474, ISSN: 2073-4859, DOI: 10.32614/RJ-2021-103 Retrieved from the Internet: URL:https://cran.r-hub.io/web/packages/bssm/vignettes/bssm.pdf> * the whole document * | 1-14 | |
| A | SIDDIQUE NAHIAN ET AL: "U-Net and Its Variants for Medical Image Segmentation: A Review of Theory and Applications", IEEE ACCESS, IEEE, USA, vol. 9, 3 June 2021 (2021-06-03), pages 82031-82057, XP011860093, DOI: 10.1109/ACCESS.2021.3086020 [retrieved on 2021-06-09] * the whole document * | 12 | |

**TECHNICAL FIELDS SEARCHED (IPC)**

G16H
A61B
G06T
G06N

The present search report has been drawn up for all claims

| Place of search | Date of completion of the search | Examiner |
|---|---|---|
| Munich | 14 December 2023 | Hilbig, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)